# EUROPEAN PATENT APPLICATION

(11) **EP 4 603 088 A1**
(43) Date of publication of application: **20.08.2025**
(21) Application number: 25156656.8
(22) Date of filing: 07.02.2025
(51) Int. Cl.: A61K 31/164, A61K 9/00, A61K 9/107, A61K 9/48, A61K 31/5415, A61P 29/00

(54) **N-PALMITOYLETHANOLAMIDE FOR USE IN COMBINATION WITH MELOXICAM IN THE TREATMENT OF INFLAMMATORY PAIN**

(30) Priority: 15.02.2024 IT 202400003214
(71) Applicant: EPITECH GROUP S.p.A., 20144 Milano (MI) (IT)
(72) Inventor: DELLA VALLE, Maria Federica, I-20144 Milano (IT); DELLA VALLE, Raffaella, I-20144 Milano (IT); MARCOLONGO, Gabriele, I-20144 Milano (IT); GHELARDINI, Carla, I-20144 Milano (IT); DI CESARE MANNELLI, Lorenzo, I-20144 Milano (IT); CUZZOCREA, Salvatore, I-20144 Milano (IT); ZUSSO, Morena, I-20144 Milano (IT)
(74) Representative: Long, Giorgio

(57) **Abstract**

The present invention relates to the use of N-palmitoylethanolamide (known as palmitoylethanolamide or PEA) in combination with meloxicam in the treatment of inflammatory pain.

In particular, the present invention is directed to palmitoylethanolamide for use in the treatment of inflammatory pain, in particular non-neuropathic inflammatory pain, where palmitoylethanolamide is administered in association or combination with meloxicam, where said administration is separate, combined, or simultaneous.

## Description

### Technical field of the invention

The present invention relates to the use of N-palmitoylethanolamide (known as palmitoylethanolamide or PEA) in combination with meloxicam in the treatment of inflammatory pain.

### Background art

Pain is one of the most common human health problems. Moreover, in recent years there has been growing attention to the recognition and management of pain also in pets, and in particular cats and dogs. In fact, based on the latest definition of pain issued by the *International Association for the Study of Pain* (IASP), to experience pain it is not necessary to know how to express it verbally, which fully includes even non-verbal subjects (such as animals) among beings capable of experiencing pain.

Based on duration, pain is classified as acute, persistent or chronic; based on origin, it is defined as nociceptive, inflammatory, or neuropathic. In conditions of tissue damage, inflammation is responsible for the production and release of mediators involved in the enhancement of pain, which in this case is defined as inflammatory.

Arachidonic acid derivatives cover an important role among these mediators: in fact, prostaglandins, prostacyclins and thromboxanes (prostanoids), produced by the action of the enzymes cyclooxygenase (COX)-1 (constitutive isoform) and COX-2 (inducible isoform), are involved in the hyper-excitation of nociceptors resulting in the appearance of allodynia.

Non-steroidal anti-inflammatory drugs or NSAIDs are the most widely used medicines in treating inflammatory pain, by virtue of the inhibitory mechanism thereof on COX. Meloxicam is one of the most common NSAIDs and is used for treating inflammatory pain, both in the human and veterinary context.

Meloxicam has a relative selectivity of action on COX-2 and belongs to that subgroup of NSAIDs called "non-coxib COX-2 selective NSAIDS". It is used for the short-term treatment of osteoarthrosis exacerbations and in the long-term treatment of pain associated with rheumatoid arthritis and ankylosing spondylitis symptoms. It is marketed in the form of different pharmaceutical formulations and it is recommended not to exceed 15 mg per day. It is also widely used in veterinary medicine, especially in treating arthrosis and post-operative pain in cats and dogs.

Although widely used in the management of inflammation and pain, meloxicam exerts serious side effects which, even today, are a cause for concern. In fact, the inhibition of prostaglandin production leads to the development of adverse events of a dose-dependent nature, with renal and hepatic toxicity, cardiovascular events, hypertension and gastrointestinal complications, especially in frail patients such as the elderly, renal patients or those undergoing multiple therapies.

The need is therefore felt to have effective and safe therapies for the correct management of inflammatory pain, both acute and chronic. To this end, it is desirable to be able to reduce the effective dosage of meloxicam.

N-palmitoylethanolamide (simply known as palmitoylethanolamide or PEA), a palmitic acid amide normally found in animal tissues, where it is produced on demand under damaging conditions, is known to carry out anti-inflammatory and anti-nociceptive actions. Preclinical and clinical studies have demonstrated the efficacy of the administration of PEA, especially in micronized form (particle size between 0.2 and 10 µM), in different types of inflammation and pain.

The analgesic effect thereof has also been compared with that of NSAIDs, and more in particular ibuprofen and celecoxib, in patients with temporomandibular pain and chronic pelvic pain. Furthermore, one study demonstrated the effect of continuous administration for 2 weeks of a combination of PEA and celecoxib on temporomandibular pain, without, however, comparing the effect with single treatments. It should be taken into account that PEA has an excellent safety profile and is free of acute and sub-chronic toxicity at least up to 1000 mg/kg daily (when administered in ultra-micronized form, Nestmann Food Sci Nutr. 2016 Jun 15;5(2):292-309).

The joint use of PEA and paracetamol, an antipyretic and analgesic drug which does not belong to the NSAID category and acts by means of mainly central mechanisms, was investigated, highlighting the benefit thereof under conditions of neuropathic pain, both experimentally caused and spontaneous. This is probably due to the fact that PEA, in particular in ultra-micronized form, also has a strong anti-inflammatory effect at the level of the central nervous system.

However, it is important to emphasize that the therapeutic improvement due to the combined use of different analgesic drugs is not obvious, as demonstrated by the fact that the association between NSAIDs and paracetamol does not entail any additional benefit.

Continuous and prolonged prophylactic use (three months) of ultra-micronized PEA, combined with the administration on demand of one or more NSAIDs (ibuprofen, diclofenac or nimesulide) was advantageous with respect to the use on demand of NSAIDs alone, in patients with migraine with and without aura. The advantage occurred from the second or third month of treatment with PEA and affected patients with migraine, considered a "syndrome" associated with central alterations typical of neuropathic pain. However, the existence of synergistic interactions between PEA and NSAIDs has not been demonstrated.

In conclusion, the prior art has not described or suggested the treatment as needed with PEA in association with an NSAID, in particular in non-neuropathic inflammatory pain conditions.

More in particular, the combination of PEA with meloxicam has not been described nor suggested in the prior art.

### Summary of the invention

The present invention derives from the surprising finding that palmitoylethanolamide (PEA), preferably if used in ultra-micronized form, when administered in combination with meloxicam, exhibits a synergistically relevant effect in treating inflammatory pain, in particular non-neuropathic inflammatory pain, resulting in a benefit for patient safety, in terms of lower incidence and severity of dose-dependent side effects, typical of this drug. In particular, the synergistic effect between PEA and meloxicam in treating inflammatory pain in humans and animals allows enhancing the anti-inflammatory effects of meloxicam, reducing the active dosage thereof.

Therefore, the present invention relates to palmitoylethanolamide for use in the treatment of inflammatory pain, in particular non-neuropathic inflammatory pain, where palmitoylethanolamide is administered in association or combination with meloxicam, where said administration is separate, combined, or simultaneous.

The invention further relates to a composition containing palmitoylethanolamide and meloxicam, in particular when usable in the treatment of inflammatory pain.

These and further objects, as outlined in the appended claims, will be described in the following description. The text of the claims should be considered included in the description in order to assess the sufficiency of description.

Further features and advantages of the invention will become apparent from the following description of preferred embodiments, given by way of non-limiting indication.

### Brief description of the drawings

Figure 1 shows the inflammatory pain tolerance threshold in response to the various treatment groups indicated in the legend, measured in the paw pressure test;
Figure 2 shows the comparison between the mean values of AUC ± SEM, obtained starting from the data shown in Figure 1;
Figure 3 shows a particle size distribution curve of ultra-micronized PEA according to an embodiment, obtained by the laser scattering method described below;
Figure 4 shows the effect of the treatments on the pain perceived 3 hours after the sub-plantar injection of CAR (Von Frey test) * p < 0.01 vs vehicle; § p < 0.05 and §§ p < 0.01 vs PEA; # p < 0.05 vs meloxicam (low);
Figure 5 shows the average analgesic power detected in the various treatment groups tested in experiment 3 (pain induced by LPS).

### Detailed description of the invention

In a first aspect, the present invention relates to palmitoylethanolamide for use in the treatment of inflammatory pain, in particular non-neuropathic inflammatory pain, where palmitoylethanolamide is administered in association or combination with meloxicam, where said administration is separate, combined, or simultaneous.

In a second aspect, the invention relates to palmitoylethanolamide for use in the treatment of inflammatory pain, in particular non-neuropathic inflammatory pain, where palmitoylethanolamide is administered as needed in association or combination with meloxicam, where said administration is separate, combined, or simultaneous.

The terms "in association" or "in combination" mean both a combination therapy and a therapy in which PEA and meloxicam are contained in a single dosage form.

The term "as needed" means an administration, also known as "on demand", which can include a single dose or several doses and which can comprise a single administration or several administrations within a time interval between a day and a week and which includes the administration of PEA and meloxicam after the onset of non-neuropathic inflammatory pain. Such a term must be understood to exclude continuous preventive, prophylactic, or curative administration.

The term "continuous administration" means an administration of several doses of the drug for a time greater than one week, more typically greater than one month.

"Separate" administration means an administration of PEA and meloxicam, administered at different times ranging from 1 minute to several hours, for example 8, 12 or 14 hours apart.

"Combined" administration means an administration of PEA and meloxicam contained in a single dosage form, i.e., a pharmaceutical or veterinary composition or formulation.

"Simultaneous" administration means an administration of PEA and meloxicam in separate dosage forms, but administered simultaneously, i.e., within a separation time between the PEA and meloxicam administrations, or vice versa, not exceeding 1 minute.

Palmitoylethanolamide can be administered in any form, for example in a non-micronized form, in a micronized form or in an ultra-micronized form.

The term "palmitoylethanolamide (or PEA) in a non-micronized form" means PEA having a particle size distribution, defined as a percentage by volume and measured with the laser light scattering method, represented by a distribution curve having the mode above 10 microns, preferably above 20 microns.

The term "palmitoylethanolamide (or PEA) in a micronized form" means PEA having a particle size distribution, defined as a percentage by volume and measured with the laser light scattering method, represented by a distribution curve having the mode between 6 microns and 10 microns.

The term "palmitoylethanolamide (or PEA) in an ultra-micronized form" means PEA having a particle size distribution, defined as a percentage by volume and measured with the laser light scattering method, represented by a distribution curve having the mode below 6 microns and above 0.5 microns.

Preferably, PEA is in an ultra-micronized form.

In an embodiment, PEA in an ultra-micronized form has a particle size distribution as defined above, measured with a Malvern Mastersizer 3000 instrument with Fraunhofer calculation algorithm, where at least 90% by volume, more preferably at least 95% by volume, of particles has a particle size of less than 6 microns (d90= 6 micron).

In a particularly preferred embodiment, PEA in an ultra-micronized form has a particle size distribution as defined above, measured with a Malvern Mastersizer 3000 instrument with Fraunhofer calculation algorithm, having a mode between 2 and 4 microns and having 100% by volume of particles smaller than 10 microns and at least 60% by volume of particles smaller than 3 microns.

It must be considered that, as defined in the European Pharmacopoeia (section 2.9.31), the particle size measurements carried out with the laser light diffraction method and expressed as d90 (maximum size of 90% by volume of the particles present in a sample) have a variability of ±15% for d90 greater than 10 microns and ±30% for d90 less than 10 microns. This means that a d90 of 6 microns measured with said method is to be understood in practice as being contained in a range between 4.2 and 7.8 microns. In other words and by way of example, a d90= 7 microns measured by laser light diffraction on a sample falls within the definition of d90= 6 microns as defined in the present patent application.

The micronization can be carried out in a fluid jet system (for example, Jetmill^{®} model system) which operates with spiral technology with a compressed air or nitrogen jet capable of exploiting kinetic energy - instead of mechanical energy - to crush the particles. Such apparatuses are conventional and will therefore not be further described, except in relation to the following features:
- Internal diameter of the micronization chamber about 300 mm;
- Fluid jet pressure 10-12 bar;
- Product supply 9-12 kg/h.

The present invention further relates to a composition comprising palmitoylethanolamide and meloxicam. Preferably, the composition of the invention consists of a dry mixture of palmitoylethanolamide/meloxicam. More preferably, palmitoylethanolamide is in micronized (m-PEA) or ultra-micronized (um-PEA) form, even more preferably palmitoylethanolamide is um-PEA, or is a mixture of at least two choices from um-PEA and/or m-PEA and/or non-micronized PEA.

Whether administered separately or combined in a single formulation, PEA and meloxicam are administered in a PEA/meloxicam weight ratio between 20:1 and 1:1, preferably between 12:1 and 5:1. More in particular, when PEA is in ultra-micronized form, the PEA/meloxicam weight ratio will preferably be between 11:1 and 3:1, more preferably between 10:1 and 5:1. When PEA is in micronized or non-micronized form, the PEA/meloxicam weight ratio will preferably be between 20:1 and 5:1, more preferably between 18:1 and 10:1.

Based on such weight ratios, for which a significant synergistic effect has been highlighted, the minimum daily dose of PEA, both in a combination therapy and in a PEA/meloxicam composition, will be at least between 2.5 mg/day and 120 mg/day. Preferably, in the case of using um-PEA, the minimum daily dose of um-PEA will be between 4 mg/day and 66 mg/day, while in the case of using non-micronized PEA or m-PEA the minimum daily dose will be between 5 mg/day and 120 mg/day.

Such doses may vary depending on the subject and in particular if the subject is of child, adult or elderly age.

Taking into account that, as widely known in the literature, PEA has a low toxicity, it will be possible to use higher doses of PEA than those described above, which were sufficient to obtain a synergistic effect on non-neuropathic inflammatory pain.

The additional PEA with respect to the amount of synergistic PEA with meloxicam can also be present in a different form than that used in association with meloxicam. For example, if PEA is in the form of um-PEA, the additional PEA may be either um-PEA, m-PEA or non-micronized PEA, or vice versa.

Therefore, the overall daily dose of PEA administered to a subject, either in the form of combination therapy or in the aforesaid composition with meloxicam, can be between 200 and 2000 mg/day, preferably between 300 and 1500 mg/day or between 400 and 1200 mg/day.

Such daily doses can be divided into dose units for an administration, for example, from 1 to 4 times a day. The dose will also depend on the route chosen for administration. It should be considered that dosage variations could be necessary depending on the patient age and weight and also on the extent of inflammatory pain to be treated. The exact dose and route of administration will ultimately be at the discretion of the attending physician.

For the purposes of the invention, PEA alone, meloxicam alone or the composition containing PEA and meloxicam can be included in pharmaceutical or veterinary formulations and can be formulated in dosage forms for oral, buccal, parenteral, rectal, topical, or transdermal administration.

For oral administration, the compounds of the invention can be found, for example, in the form of tablets or capsules, hard or soft, prepared in the conventional fashion with pharmaceutically acceptable excipients such as binders (e.g., pregelatinized cornstarch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or inhibiting agents (e.g., sodium lauryl sulfate). The tablets can be coated by methods well known in the art. The liquid preparations for oral administration can be, for example, in the form of solutions, syrups or suspensions or they can be freezedried or granulated products to be reconstituted, before use, with water or other suitable vehicles. Such liquid preparations can be prepared through conventional methods with pharmaceutically acceptable additives such as suspending agents (e.g., sorbitol syrup, cellulose derivatives, or edible hydrogenated fats); emulsifiers (e.g., lecithin or acacia); non-aqueous vehicles (e.g., almond oil, oily esters, ethyl alcohol or fractionated vegetable oils); and preservatives (e.g., methyl- or propyl-p-hydroxybenzoates, sorbic acid, benzoic acid or salts thereof). The preparation can also conveniently contain flavorings, dyes, and sweeteners.

The preparations for oral administration can be appropriately formulated to allow the controlled release of the active constituent.

For buccal administration, the compounds of the invention can be in the form of tablets or granules formulated in the conventional manner, which are suitable for an absorption at the level of the buccal mucosa. Typical buccal formulations are tablets for sublingual administration.

The compounds of the invention can be formulated for parenteral administration by injection. The injection formulations can be presented as a single dose, for example in vials, with an added preservative. The compositions can appear in such a form as suspensions, solutions, or emulsions in oily or aqueous vehicles and can contain agents of the formulation such as suspension, stabilizers and/or dispersants. Alternatively, the active ingredient or the mixture of active ingredients can be found in the form of a powder to be reconstituted, before use, with a suitable vehicle, for example with sterile water.

The compounds of the invention can also be formulated according to rectal formulations such as suppositories or retention enemas, for example containing the basic components of the common suppositories such as cocoa butter or other glycerides.

In addition to the formulations described above, the compounds of the invention can also be formulated as a deposit preparation for administration over a day to a week. Such long-acting formulations can be administered by implantation (e.g., subcutaneously, transcutaneously or intramuscularly) or intramuscular injection. Therefore, for example, the composition can be formulated with appropriate polymer or hydrophobic materials (for example in the form of an emulsion in a suitable oil) or ion exchange resins or as minimally soluble derivatives.

The compounds or composition of the invention can also be administered in the form of oral sprays or nasal sprays.

The invention further relates to a composition comprising or consisting of a mixture of palmitoylethanolamide, preferably ultra-micronized palmitoylethanolamide, meloxicam and pharmaceutically acceptable excipients, where the PEA/meloxicam weight ratio is between 20:1 and 1:1, preferably between 12:1 and 5:1, and where PEA is contained in an amount between 200 and 2000 mg.

In certain embodiments, the aforesaid composition, or palmitoylethanolamide mixture for separate or sequential administration, further comprises 2-pentadecyl-2-oxazoline (also referred to as PEA-OXA) which is a PEA analog.

PEA can also be administered in the form of dietary compositions, food supplements, complementary feed, and foods for special medical purposes (FSMP).

The term "foods for special medical purposes" means products authorized according to (EU) regulation 2016/128. Such a term refers to a product to be administered under medical supervision, thus assimilating such an FSMP to a drug.

The formulations according to the invention can be prepared according to conventional methods, such as those described in Remington's Pharmaceutical Sciences Handbook, Mack Pub. Co., N.Y., USA, 17th edition, 1985 o in Remington, The Science and Practice of Pharmacy, Edited by Allen, Loyd V., Jr, 22nd edition, 2012 or in subsequent editions.

### EXPERIMENTAL SECTION

### Micronization procedure

PEA was micronized as previously described.

The ultra-micronization was carried out in a fluid jet system (in particular, the Jetmill^{®} model system) which operates with compressed air jet "spiral technology".

### Optimal micronization conditions:

- internal diameter of the micronization chamber 300 mm;
- fluid jet pressure 8 bar;
- product supply 9-12 kg/h.

### Determination of the particle size distribution

The determination of the particle size distribution was carried out on a wet sample, after 1-minute sonication.

A Malvern Mastersizer 3000 instrument operating with the LALLS (Low Angle Laser Light Scattering) technique and a Fraunhofer calculation algorithm was used.

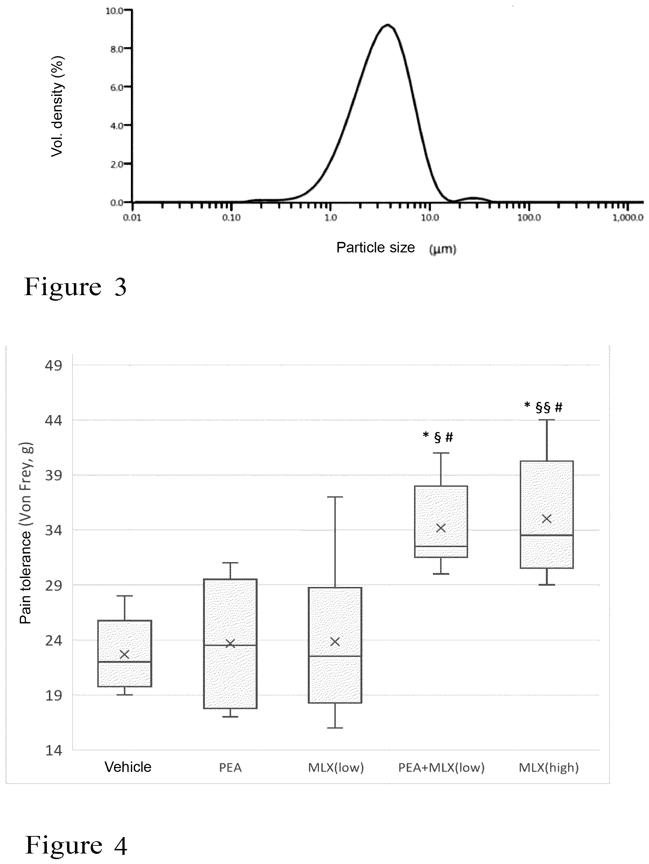

The particle size distribution graph is shown in Figure 3.

### Biological experimentation

EXPERIMENT 1 - Efficacy and synergy of PEA and Meloxicam in an animal model of CFA-induced inflammatory pain

In the *in vivo* experimentation, male Sprague-Dawley rats (200-250 g) (Envigo, Varese, Italy) fed "ad libitum" and housed in cages with a controlled sleep/wake cycle were used.

Before the start of the study, the animals were subjected to a 1-week acclimatization period at the Center for Laboratory Animal Sheltering (Ce.S.A.L.) of the University of Florence, following all experimental procedures and protocols compliant with the principles of care and welfare of laboratory animals approved by the Italian Ministry of Health (Italian Legislative Decree 2014/26), European directives (EU Directive 2010/63) and the ARRIVE guidelines.

In order to cause inflammatory pain, Complete Freund's Adjuvant (CFA) was injected at a rate of 50 µL into the joint cavity of the left leg between the tibiofibular and tarsal bones after light anesthesia with 2% isoflurane. The control group was subjected to the same procedure, injecting an equal volume of saline solution (vehicle).

The animals were divided into 6 groups of 6 rats each and treated acutely *per* os with a single administration, starting from the seventh day after the induction of joint damage.
- Group 1: healthy rats subjected to intra-articular injection of saline (vehicle group)
- Group 2: rats injected with CFA and treated with 1% CMC, corresponding to the vehicle in which the compounds for the treatments of the following groups were suspended (CFA group);
- Group 3: rats injected with CFA and treated with micronized PEA 10 mg/kg;
- Group 4: rats injected with CFA and treated with meloxicam 30 mg/kg;
- Group 5: rats injected with CFA and treated with meloxicam 3 mg/kg;
- Group 6: rats injected with CFA and treated with meloxicam 3 mg/kg + micronized PEA 10 mg/kg.

The micronized PEA used in the experiments (hereinafter referred to as PEA for simplicity) had an average particle size between 0.2 and 10 µM and a d90 of about 6 microns. All animals were subjected to the paw pressure test at the level of the ipsilateral paw, performed before (T0) or after 15, 30, 45 and 60 minutes after the single administration of the treatment (T15, T30, T45 and T60, respectively). In particular, the nociceptive threshold was calculated with an analgesiometer (Ugo Basile, Varese, Italy) by applying an increasing pressure at constant speed (32 g/s) by means of a non-pointed conical support on the dorsal surface of the ipsilateral paw with respect to the injection of CFA (or vehicle). The nociceptive threshold was expressed as the force (in decagrams, dag) to which the animal reacts by retracting the paw or vocalizing (Leighton GE et al. kappa-Opioid agonists produce antinociception after i.v. and i.c.v. but not intrathecal administration in the rat. Br J Pharmacol. 1988; 93: 553-60).

### Statistical analysis

The values emerged from the paw pressure test in the different treatment groups were compared with each other using the Generalized Linear Mixed Model (GLMM) followed by post-hoc analysis based on Tukey-Kramer correction for multiple comparisons, referring to the single treatment group, considering all the times. For the synergy analysis, the area under the curve (AUC) was considered, calculated with the trapezoid rule for the following four treatment groups:
CFA + vehicle
CFA + PEA 10 mg/kg
CFA + meloxicam 3 mg/kg
CFA + PEA (10 mg/kg) + meloxicam (3 mg/kg)
in order to verify whether the effect of the combined administration of PEA and meloxicam was greater than the sum of the individual effects of the two treatments used individually.

The AUC was analyzed by a two-way factor analysis of variance (2x2 ANOVA), after plotting the mean values, according to the procedure shown by Slinker BK. The statistics of synergism. J Mol Cell Cardiol. 1998 Apr;30(4):723-31. The values were expressed as mean ± standard error mean (SEM). All the statistical analyses were carried out using the software SAS, version 9.4 (SAS Institute, Cary, NC, USA). The value p < 0.05 was considered significant.

### Results of the experiments

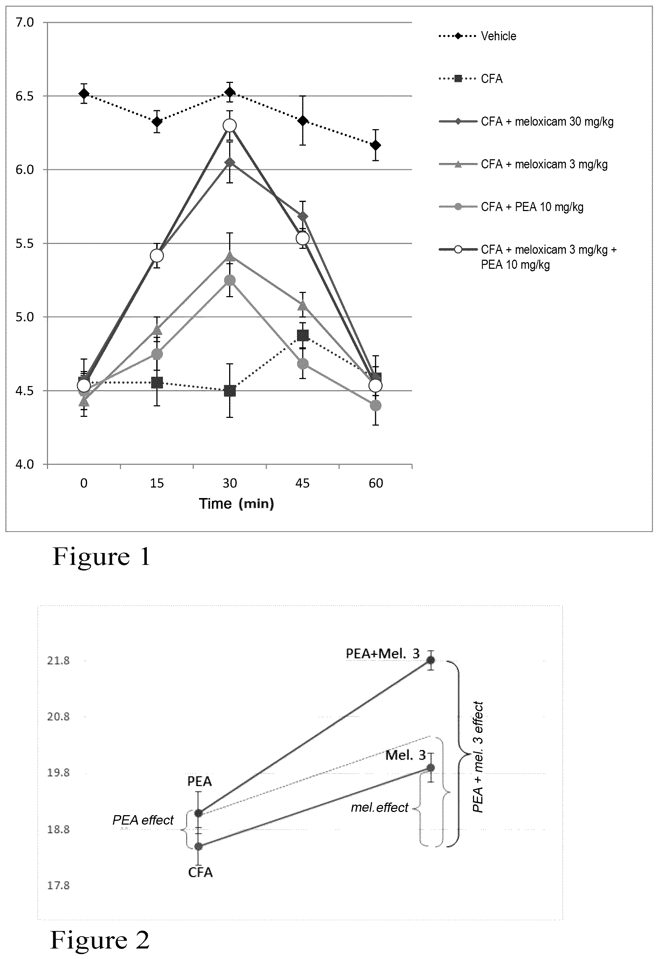

The results obtained allowed verifying that (Figure 1) :
- The injection of CFA (dotted line with squares) causes significant inflammatory pain, as shown by the significant lowering of the tolerance threshold (p<0.0001) with respect to the vehicle group (dotted line with diamonds);
- Unlike meloxicam at 3 mg/kg (line with triangles) and PEA alone (line with solid circles), which show no significant effect with respect to CFA (p = 0.1405 and p = 0.9588, respectively), meloxicam administered at 30 mg/kg (line with diamonds) significantly counteracts the threshold lowering caused by CFA (p = 0.0001);
- Surprisingly, the combined use of PEA and low-dose meloxicam (3 mg/kg), that - as mentioned above - individually had no effect, significantly reduces pain (line with empty circles; p < 0.0001 with respect to CFA) .

The experiments show that the effect produced by combining PEA with a low dose of meloxicam is not significantly different (p = 1.0000) from that obtained with meloxicam 30 mg/kg, which means that the addition of PEA to meloxicam allows obtaining the same analgesic effect for 10-fold lower dosages of meloxicam.

The synergy analysis carried out on the AUC data allowed confirming a synergistic effect between PEA and low-dose meloxicam, both visually (Figure 2), and based on the probability which emerged from the Anova 2x2 analysis (Table 1):

**Table 1 - Results of the two-way factor analysis of variance (ANOVA 2x2) carried out on the data plotted in Figure 2 (PEA= um-PEA 10 mg/kg; Mel3= meloxicam 3 mg/kg)**

| Source | DF | Sum of Squares | Mean Squares | F Value | Pr > F |
|---|---|---|---|---|---|
| Mel 3 | 1 | 24.6139 | 24.6139 | 48.1 | <0.0001 |
| PEA | 1 | 9.5067 | 9.5067 | 18.58 | 0.0003 |
| **PEA*Mel 3** | **1** | **2.3406** | **2.3406** | **4.57** | **0.0450** |
| Error | 20 | 10.2342 | 0.5117 | | |

### EXPERIMENT 2 - Efficacy and synergy of PEA and Meloxicam in an animal model of inflammatory pain induced by Carrageenan

The study was conducted on male Sprague-Dawley rats and inflammatory pain was induced with sub-plantar injection of saline solution containing 1% carrageenan (CAR). The animals were subsequently divided into 5 groups of 6 rats each. All treatments were carried out orally, 30 minutes before the induction of inflammatory pain.
Group 1: CAR rats + carboxymethyl cellulose (CMC) 1%, vehicle used to suspend molecules (vehicle);
Group 2: CAR rats + PEA 3 mg/kg;
Group 3: CAR rats + MLX 0.05 mg/kg (MLX_low);
Group 4: CAR rats + PEA combination 3 mg/kg + MLX 0.05 mg/kg (PEA+ MLX_low);
Group 5: CAR rats + MLX 0.2 mg/kg (MLX_high).

The PEA used in the experiment had an average particle size between 0.2 um and 10 um and a d90 of approximately 6 um. The retraction of the paw (expressed in grams, g) as a function of the pressure exerted with the Von Frey filaments was measured with an analgesimeter (Ugo Basile, Comerio, Varese, Italy), in each treatment group, before the sub-injection plantar of the CAR and after 3h and 5h.

### Statistical analysis

Pain tolerance measured with Von Frey filaments in different treatment groups was analyzed by Generalized Linear Model (GLM) followed by post-hoc analysis based on Tukey-Kramer correction for multiple comparisons. In order to verify whether the association of PEA and MLX exerted a synergistic effect (i.e., the combined administration of PEA and MLX produced an effect greater than the sum of the individual effects of the two treatments used individually), factorial analysis of variance was used (ANOVA 2x2), according to the procedure illustrated by Slinker BK (The statistics of synergism. J Mol Cell Cardiol. 1998;30(4):723-31). The results of the analyses were expressed as mean ± standard error of the mean (SEM). All analyses were performed using SAS software, version 9.4 (SAS Institute, Cary, NC, USA). P value < 0.05 was considered significant.

### Results of the experiments

In the group treated with PEA alone or with low-dose MLX alone, no analgesic effect significantly different from the vehicle was observed either 3 or 5 hours after the sub-plantar injection of CAR. Conversely, the combination of PEA and low-dose MLX was effective in counteracting inflammatory pain at both 3 and 5 hours (p=0.0084 and p=0.0004 vs vehicle, respectively). Not only that, but the efficacy of this combination was found to be almost identical to that of high-dose MLX (p=0.99 both 3 and 5 hours after CAR injection), demonstrating the fact that the addition of an inactive dose of PEA at a low (and inactive) dose of MLX allows to obtain the same analgesia that is observed using MLX at a 4 times higher dosage. Figure 4 shows the observed situation.

The 2x2 factorial analysis demonstrated the synergistic effect between PEA and MLX, both 3 and 5 hours after CAR injection. The probability that the effect of the combination was greater than the sum of the effects of the individual substances was, in fact, less than 0.05 at both times (p=0.0441 and p=0.0203, at 3 and 5 hours respectively, as reported in Table 2 and Table 3):

**Table 2 - Result of the 2x2 ANOVA analysis 3 hours after CAR injection**

| **3h** | DF | Sum of Squares | Mean Squares | F Value | Pr > F |
|---|---|---|---|---|---|
| Source | | | | | |
| PEA | 1 | 192,7 | 192,7 | 6,80 | 0,0168 |
| MLX | 1 | 204,2 | 204,2 | 7,21 | 0,0142 |
| **PEA*MLX_low** | 1 | 130,7 | 130,7 | 4,61 | **0,0441** |
| Error | 20 | 566,3 | 28,3 | | |

**Table 3 - Result of the 2x2 ANOVA analysis 5 hours after CAR injection**

| **5h** | DF | Sum of Squares | Mean Squares | F Value | Pr> F |
|---|---|---|---|---|---|
| Source | | | | | |
| PEA | 1 | 266,7 | 266,7 | 11,31 | 0,0031 |
| MLX | 1 | 253,5 | 253,5 | 10,75 | 0,0038 |
| **PEA*MLX_low** | 1 | 150,0 | 150,0 | 6,36 | **0,0203** |
| Error | 20 | 471,7 | 23,6 | | |

### EXPERIMENT 3 - Efficacy and synergy of PEA and Meloxicam in an animal model of LPS-induced inflammatory pain

Male Sprague-Dawley rats were housed in groups of four, in 26x41 cm cages, at a temperature of 23±1°C, with a 12-hour circadian cycle, and water/food "ad libitum" (standard diet). LPS (10 µg in 50 µl of 0.9% NaCl, Sigma-Aldrich) was injected into the plantar surface of the hind paw of the rat following isoflurane anesthesia. Control animals were treated with vehicle alone (0.9% NaCl). Ten minutes before the LPS injection, the compounds to be tested or the vehicle alone (1% CMC) were administered orally, according to the following treatment groups (N=8 where not otherwise specified):
CTRL (healthy animals injected with 0.9% NaCl)
VEIC (animals injected with LPS and treated with vehicle, N=9)
MLX30 (animals injected with LPS and treated with meloxicam 30 mg/kg)
MLX3 (animals injected with LPS and treated with meloxicam 3 mg/kg)
PEA10 (animals injected with LPS and treated with PEA 10 mg/kg)
PEA10+MLX3 (animals injected with LPS and treated with PEA 10 mg/kg + meloxicam 3 mg/kg)
PEA5 (animals injected with LPS and treated with PEA 5 mg/kg)
PEA5 + MLX3 (animals injected with LPS and treated with PEA 5 mg/kg + meloxicam 3 mg/kg).

PEA used in the experiment had an average particle size between 0.2 and 10 um and a d90 of approximately 6 microns. Inflammatory pain induced by LPS injection was measured by paw pressure test. Briefly, before and sixty min after LPS injection, increasing pressure at a constant rate (32 g/s) was applied to the ipsilateral paw of the rat using a non-sharp conical support. The nociceptive threshold has been expressed as the force to which the animal reacts by retracting its paw or vocalizing (Leighton et al., Br J Pharmacol 93:553-560,1988). An analgesimeter (Ugo Basile, Varese) was used for this purpose. The results were expressed in terms of analgesic potency, defined as percentage inhibition (mean ± SEM) of inflammatory pain induced by LPS and calculated according to the formula: 100*(treatm-VEIC)/(CTRL-VEIC).

### Statistical analysis

The results were analyzed via Generalized Linear Model (GLM) followed by post-hoc analysis based on Tukey-Kramer correction for multiple comparisons. The results of the analyses were expressed as mean ± standard error of the mean (SEM). In order to verify whether the effect of PEA and meloxicam was synergistic, it was analyzed whether the extent of the inhibition of inflammatory pain by LPS was greater for the association of the two substances compared to the substances used individually. To this end, factorial analysis of variance (ANOVA 2x2) according to Slinker BK (1998) was used. All analyses were performed using SAS software, version 9.4 (SAS Institute, Cary, NC, USA). P value < 0.05 was considered significant.

### Results of the experiments

Sixty minutes after LPS injection, the nociceptive threshold dropped from 66.7±1.2 g (control group) to 38.1±1.3 g (p<0.0001). Treatment with meloxicam (both at 3 mg/kg and 30 mg/kg) and with PEA (both at 5 mg/kg and 10 mg/kg, alone or associated with low-dose meloxicam) significantly counteracted the pro-algic effect of LPS (p<0.0001 for all comparisons).

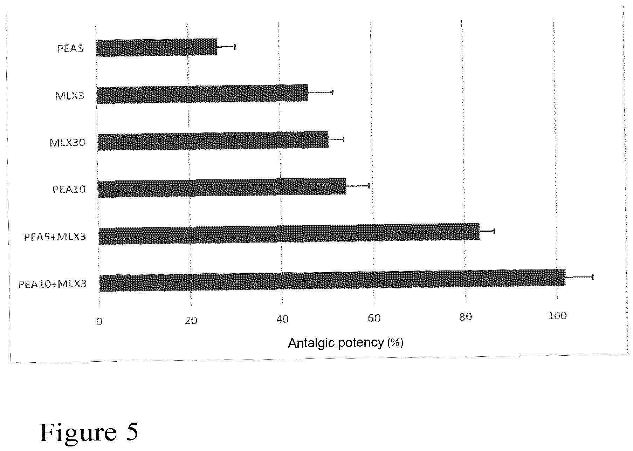

As illustrated in Figure 5, the analgesic power of the tested treatments was between 26.5% and 101.8%, with the following order of magnitude PEA10+MLX3 > PEA5+MLX3 > PEA10 > MLX30 > MLX3 > PEA5. In particular, the association between PEA (both at 5 mg/kg and 10 mg/kg) and meloxicam (3 mg/kg) appeared to exert a more powerful analgesic effect than all other treatments, as can be seen from the comparison between groups reported in Table 4:

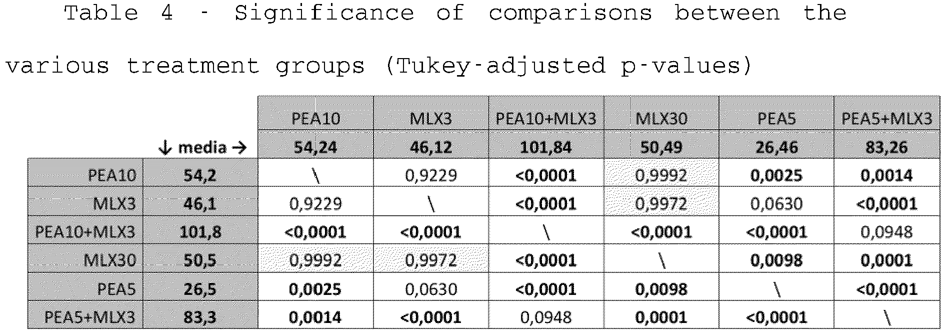

Surprisingly, the nociceptive threshold detected at the end of the experiment (60 min) in the PEA10+MLX3 group was almost identical to that measured at the same time in the healthy control group (67.2±1.7 g vs 66.7±1. 2 g; p= 0.9998), confirming the ability of the tested association to completely reverse the lowering of the nociceptive threshold induced by LPS, inhibiting the development of inflammatory pain. A similar situation was detected in the group treated with PEA 5 mg/kg and meloxicam 3 mg/kg (61.9±0.9 g; p = 0.0777 vs healthy control). The 2x2 factorial analysis highlighted that the addition of PEA (both at 5 mg/kg and 10 mg/kg) was able to synergistically enhance the effect of low-dose meloxicam. In particular, the analgesic potency obtained by administering the combination of PEA and MLX was significantly higher than the combination of the analgesic potency of PEA and MLX (p<0.0001 and p=0.0014, respectively for PEA at 10 mg/kg and 5 mg/kg) . The analysis report is shown in Table 5 and Table 6:

**Table 5 - Result of the 2x2 ANOVA analysis for the PEA10MLX3 association**

| | DF | Sum of Squares | Mean Squares | F Value | Pr > F |
|---|---|---|---|---|---|
| Source | | | | | |
| PEA10 | 1 | 1,34 | 1,34 | 28,92 | <0,0001 |
| MLX3 | 1 | 0,05 | 0,05 | 1,18 | 0,2863 |
| PEA10+MLX3 | 1 | 1,26 | 1,26 | 27,31 | **<0,0001** |
| Error | 28 | 1,29 | 0,05 | | |

**Table 6 - Result of the 2x2 ANOVA analysis for the PEA5MLX association**

| | DF | Sum of Squares | Mean Squares | F Value | Pr > F |
|---|---|---|---|---|---|
| Source | | | | | |
| PEA5 | 1 | 5,40 | 5,40 | 32,48 | <0,0001 |
| MLX3 | 1 | 11,06 | 11,06 | 66,5 | <0,0001 |
| PEA5+MLX3 | 1 | 2,06 | 2,06 | 12,39 | **0,0014** |
| Error | 29 | 4,83 | 0,17 | | |

In light of the results set forth above, the inflammatory pain which can be treated according to the present invention is preferably selected from:
- Pain resulting from tissue injuries;
- Post-operative pain
- Toothache;
- Pain and inflammation of oral cavity and throat;
- Muscular and rheumatic pain;
- Menstrual cramps (dysmenorrhea);
- Inflammatory pain associated with capsulitis and bursitis;
- Inflammatory pain associated with tendonitis and tenosynovitis;
- Inflammatory pain associated with osteoarthritis;
- Inflammatory pain associated with periarthritis;
- Inflammatory pain associated with rheumatoid arthritis;
- Inflammatory pain associated with ankylosing spondylitis;
- Inflammatory pain associated with acute gout.

The invention will now be further described through the following formulation examples.

### Formulation examples

um-PEA = Ultra-micronized palmitoylethanolamide
m-PEA = Micronized palmitoylethanolamide
non-m PEA = non-micronized palmitoylethanolamide
PEA-OXA = 2-pentadecyl-2-oxazoline

### Example 1 - Soft capsule for human use

| | |
|---|---|
| Bovine gelatin | 155 mg |
| um-PEA | 5 mg |
| Meloxicam | 1.5 mg |
| Glycerol | 77.6 mg |
| Sunflower oil | 124 mg |
| Medium chain triglycerides | 100 mg |
| Vegetable fat | 100 mg |
| Soy lecithin | 34 mg |
| Water | 11.6 mg |
| Glyceryl stearate | 12 mg |

### Example 2 - Soft capsule for human use

| | |
|---|---|
| Bovine gelatin | 155 mg |
| um-PEA | 10 mg |
| Meloxicam | 3 mg |
| Glycerol | 77.6 mg |
| Sunflower oil | 124 mg |
| Medium chain triglycerides | 100 mg |
| Vegetable fat | 100 mg |
| Soy lecithin | 34 mg |
| Water | 11.6 mg |
| Glyceryl stearate | 12 mg |

### Example 3 - Hard capsule for human use

| | |
|---|---|
| Acid-resistant vegetable gelatin capsule, "0" format PEA-OXA | 300 mg |
| um-PEA | 10 mg |
| Meloxicam | 3 mg |
| Corn dextrin | 100 mg |
| Silicon dioxide | 10 mg |

### Example 4 - Gastro-resistant tablet

| | |
|---|---|
| m-PEA | 600 mg |
| um-PEA | 10 mg |
| Meloxicam | 3 mg |
| Dextrose | 52 mg |
| Rice starch | 120 mg |
| Microcrystalline cellulose | 120 mg |
| Polysorbate 80, plant origin | 8 mg |
| Silicon dioxide | 10 mg |
| Gastro-resistant coating | 40 mg |

### Example 5 - Orosoluble granules

| | |
|---|---|
| m-PEA | 600 mg |
| um-PEA | 5 mg |
| Meloxicam | 1.5 mg |
| Sorbitol | 180 mg |
| Fructose | 180 mg |
| Polysorbate | 8010 mg |
| PVP K | 3010 mg |
| Sucrose Palmitate | 20 mg |

### Example 6 - Effervescent tablet

| | |
|---|---|
| m-PEA | 400 mg |
| um-PEA | 10 mg |

| | |
|---|---|
| Meloxicam | 3 mg |
| Corn dextrin | 500 mg |
| Potassium bicarbonate | 343 mg |
| Potassium carbonate | 108 mg |
| Anhydrous citric acid | 384 mg |
| Fructose | 130 mg |
| Polysorbate 80 | 15 mg |
| Lemon flavoring | 10 mg |

### Example 7 - Pediatric chewable tablet

| | |
|---|---|
| m-PEA | 300 mg |
| um-PEA | 10 mg |
| Meloxicam | 1.5 mg |
| Sorbitol | 81.5 mg |
| Fructose | 220 mg |
| Croscarmellose | 50 mg |
| Citric acid | 22.4 mg |
| Flavoring | 6 mg |
| Magnesium stearate | 9 mg |
| Silicon dioxide | 9 mg |
| Sucrose palmitate | 6 mg |
| Dibasic calcium phosphate dihydrate | 94.1 mg |
| Microcrystalline cellulose | 100 mg |

### Example 8 - Pediatric suspension, 100 ml multi-dose bottle

| | |
|---|---|
| m-PEA | 4.0 g |
| um-PEA | 100 mg |

| | |
|---|---|
| Meloxicam | 30 mg |
| Sucrose | 30.0 g |
| Sodium carboxymethyl cellulose | 0.65 g |
| Microcrystalline cellulose | 1.35 g |
| Polysorbate 80 | 0.1 g |
| Potassium Sorbate | 0.1 g |
| Benzoic Acid | 0.075 g |
| Citric acid | 0.15 g |
| Flavoring | 0.1 g |
| Water q.s. to 100 ml | |

### Example 9 - Oral suspension, 300 ml multi-dose bottle (1 dose = 10 ml)

| | |
|---|---|
| m-PEA | 36 g |
| um-PEA | 300 mg |
| Meloxicam | 90 mg |
| Corn dextrin | 75 g |
| Sodium carboxymethyl cellulose | 1.5 g |
| Microcrystalline cellulose | 4.5 g |
| Sucrose palmitate | 0.3 g |
| Potassium Sorbate | 0.3 g |
| Benzoic Acid | 0.225 g |
| Citric acid | 0.25 g |
| Flavoring | 0.3 g |
| Steviol glycosides | 0.045 g |
| Water q.s. to 300 ml | |

### Example 10 - 2.0 g Suppositories

| | |
|---|---|
| Suppository base (C10-18 Triglycerides-Polysorbate 65) | 1695 mg |
| non-m PEA | 300 mg |
| um-PEA | 10 mg |
| Meloxicam | 3 mg |

### Example 11 - Veterinary quadrisect tablet

| | |
|---|---|
| m-PEA | 600 mg |
| um-PEA | 10 mg |
| Meloxicam | 3 mg |
| Palatability enhancer F20729 | 85 mg |
| Microcrystalline cellulose | 140 mg |
| Croscarmellose | 54 mg |
| Glyceryl Dibehenate | 90 mg |
| Hydroxypropyl cellulose | 20 mg |
| Polysorbate 80 | 5 mg |
| Magnesium Stearate | 6 mg |

### Example 12 - Gastro-resistant tablet

| | |
|---|---|
| PEA-OXA | 600 mg |
| um-PEA | 10 mg |
| Meloxicam | 3 mg |
| Dextrose | 52 mg |
| Rice starch | 120 mg |
| Microcrystalline cellulose | 120 mg |
| Polysorbate 80, plant origin | 8 mg |
| Silicon dioxide | 10 mg |
| Gastro-resistant coating | 40 mg |

### Example 13 - Combined formula

### Blister A - PEA tablets

| | |
|---|---|
| m-PEA | 600 mg |
| um-PEA | 10 mg |
| Dextrose | 52 mg |
| Rice starch | 120 mg |
| Microcrystalline cellulose | 120 mg |
| Polysorbate 80, plant origin | 8 mg |
| Silicon dioxide | 10 mg |

### Blister B - Meloxicam tablets

| | |
|---|---|
| Meloxicam | 3 mg |
| Dibasic calcium phosphate dihydrate | 40 mg |
| Magnesium stearate | 5 mg |
| Glyceryl dibehenate | 10 mg |
| Sorbitol | 50 mg |
| Gastro-resistant coating | 10 mg |

### Example 14 - Soft capsule for human use

| | |
|---|---|
| Bovine gelatin | 237 mg |
| m-PEA | 100 mg |
| um-PEA | 10 mg |
| PEA-OXA | 50 mg |
| Meloxicam | 3 mg |
| Glycerol | 129 mg |
| Sunflower oil | 366 mg |
| Medium chain triglycerides | 51.5 mg |
| Vegetable fat | 50.8 mg |

| | |
|---|---|
| Soy lecithin | 30.0 mg |
| Water | 18.8 mg |
| Glyceryl stearate | 8.8 mg |

### Example 15 - Single-dose skin emulsion

| | |
|---|---|
| um-PEA | 10 mg |
| Meloxicam | 3 mg |
| Ethoxydiglycol | 300 mg |
| Sunflower lecithin | 30 mg |
| Caprylic capric triglycerides | 150 mg |
| Glyceryl stearate | 30 mg |
| PEG100 Stearate | 30 mg |

Occlusive adhesive polymer dressing 10x10 cm

### Example 16 - Multi-dose injectable suspension 30 ml

| | |
|---|---|
| Um-PEA | 150 mg |
| Meloxicam | 45 mg |
| Polysorbate 80 | 600 mg |

Ethoxydiglycolq.s.to 30ml

### Example 17 - Single-dose injectable solution

| | |
|---|---|
| PEA | 10 mg |
| Meloxicam | 3 mg |
| Soy lipids | 200 mg |
| Egg lecithin | 24 mg |
| Glycerol | 100 mg |

Water for injectables q.s. to 2 ml.

## Claims

1. Palmitoylethanolamide for use in the treatment of inflammatory pain, in particular non-neuropathic inflammatory pain, wherein the palmitoylethanolamide is administered in association or combination with meloxicam, wherein said administration is separate, combined, or simultaneous.

2. Palmitoylethanolamide for use according to claim 1, wherein the palmitoylethanolamide is administered as needed.

3. Palmitoylethanolamide for use according to claim 1 or 2, wherein the palmitoylethanolamide is in non-micronized form, having a particle size distribution, defined as a percentage by volume and measured by the laser light scattering method, represented by a distribution curve having the mode above 10 microns, preferably above 20 microns.

4. Palmitoylethanolamide for use according to claim 1 or 2, wherein the palmitoylethanolamide is in micronized form, having a particle size distribution, defined as a percentage by volume and measured by the laser light scattering method, represented by a distribution curve having the mode between 6 microns and 10 microns.

5. Palmitoylethanolamide for use according to claim 1 or 2, wherein palmitoylethanolamide is in ultra-micronized form having a particle size distribution, defined as percentage by volume and measured by the laser light scattering method, represented by a distribution curve having the mode below 6 microns and above 0.5 microns.

6. Palmitoylethanolamide for use according to claim 5, having a particle size distribution, defined as a percentage by volume and measured by the laser light scattering method, measured with a Malvern Mastersizer 3000 instrument with Fraunhofer calculation algorithm, wherein at least 90% by volume, preferably at least 95% by volume, of particles has a particle size less than 6 microns.

7. Palmitoylethanolamide for use according to claim 5, wherein the palmitoylethanolamide has a particle size distribution, defined as a percentage by volume and measured by the laser light scattering method, measured with a Malvern Mastersizer 3000 instrument with Fraunhofer calculation algorithm, having a mode between 2 and 4 microns and having 100% by volume of particles less than 10 microns and at least 60% by volume of particles less than 3 microns.

8. Palmitoylethanolamide for use according to any one of claims 1 to 7, wherein PEA and meloxicam are administered in a PEA/Meloxicam weight ratio between 20:1 and 1:1, preferably between 12:1 and 5:1.

9. Palmitoylethanolamide for use according to claim 8, wherein, when PEA is in ultra-micronized form, the PEA/meloxicam weight ratio will preferably be between 11:1 and 3:1, more preferably between 10:1 and 5:1, and when PEA is in micronized or non-micronized form, the PEA/meloxicam weight ratio will preferably be between 20:1 and 5:1, more preferably between 18:1 and 10:1.

10. Palmitoylethanolamide for use according to any one of claims 1 to 9, wherein the overall daily dose of PEA administered to a subject is between 200 and 2000 mg/day, preferably between 300 and 1500 mg/day, or between 400 and 1200 mg/day.

11. Palmitoylethanolamide for use according to any one of claims 1 to 10, wherein palmitoylethanolamide and meloxicam are contained in pharmaceutical or veterinary formulations and are formulated in dosage forms for oral, buccal, parenteral, rectal, topical or transdermal administration.

12. Palmitoylethanolamide for use according to any one of claims 1 to 10, wherein the palmitoylethanolamide is contained in dietary compositions, food supplements, complementary feed, or foods for special medical purposes (FSMP).

13. Palmitoylethanolamide for use according to any one of claims 1 to 12, further comprising the administration of 2-pentadecyl-2-oxazoline.

14. Palmitoylethanolamide for use according to any one of claims 1 to 13, wherein the non-neuropathic inflammatory pain is a pain selected from:
• Pain resulting from tissue injuries;
• Post-operative pain;
• toothache;
• Pain and inflammation of oral cavity and throat;
• Muscular and rheumatic pain;
• Menstrual cramps (dysmenorrhea);
• Inflammatory pain associated with capsulitis and bursitis;
• Inflammatory pain associated with tendonitis and tenosynovitis;
• Inflammatory pain associated with osteoarthritis;
• Inflammatory pain associated with periarthritis;
• Inflammatory pain associated with rheumatoid arthritis;
• Inflammatory pain associated with ankylosing spondylitis;
• Inflammatory pain associated with acute gout.

15. A composition comprising or consisting of a mixture of palmitoylethanolamide, preferably ultra-micronized palmitoylethanolamide, Meloxicam, pharmaceutically acceptable excipients, and optionally 2-pentadecyl-2-oxazoline, wherein the PEA/meloxicam weight ratio is between 20:1 and 1:1, preferably between 12:1 and 5:1, wherein PEA is contained in an amount between 200 and 2000 mg.
